Europäisches Patentamt

European Patent Office

Office européen des brevets

(11) Veröffentlichungsnummer: **0 292 828 B1**

(12) **EUROPÄISCHE PATENTSCHRIFT**

(45) Veröffentlichungstag der Patentschrift: **13.11.91**

(21) Anmeldenummer: **88107812.5**

(22) Anmeldetag: **16.05.88**

(51) Int. Cl.⁵: **G01N 13/00**, G01N 33/24, E02D 1/00

(54) **Verfahren und Vorrichtung zum Erzeugen von definierten Bodenschubspannungen.**

(30) Priorität: **27.05.87 DE 3717969**

(43) Veröffentlichungstag der Anmeldung:
**30.11.88 Patentblatt 88/48**

(45) Bekanntmachung des Hinweises auf die
Patenterteilung:
**13.11.91 Patentblatt 91/46**

(84) Benannte Vertragsstaaten:
**BE CH FR GB LI NL**

(56) Entgegenhaltungen:
**AU-B- 563 177**
**US-A- 4 537 064**

**REVIEW OF SCIENTIFIC INSTRUMENTS, Band
53, Nr. 12, Seiten 1851-1854, Dezember 1982,
New York, USA; S. R. BUSSOLARI et al.:
"Apparatus for subjecting living cells to
fluid shear stress"**

(73) Patentinhaber: **Gust, Giselher, Dr.**
**Zeppelinring 33**
**W-2300 Kiel 14(DE)**

(72) Erfinder: **Gust, Giselher, Dr.**
**Zeppelinring 33**
**W-2300 Kiel 14(DE)**

(74) Vertreter: **Schupfner, Gerhard et al**
**Patentanwälte Dipl.-Ing. Hans-Jürgen Müller**
**Dipl.-Chem.Dr.phil.nat. Gerhard Schupfner**
**Dipl.-Ing. Hans-Peter Gauger**
**Karlstrasse 5 D-2110 Buchholz in der Nord-**
**heide(DE)**

## Beschreibung

Die Erfindung betrifft ein Verfahren zum Erzeugen von definierten Bodenschubspannungen an einer Substrat/Fluid-Grenzschicht, insbesondere an einer zu untersuchenden, in einen geschlossenen Raum eingebrachten Sediment/Wasser-Grenzschicht, sowie eine entsprechende Vorrichtung.

Die Untersuchung der Sediment/Wasser-Grenzschicht von Gewässern, beispielsweise zur Bestimmung des vertikalen Austausches von chemischen Substanzen zwischen dem Boden und dem Wasser oder der Konzentrationsprofile sowie der Reservoir-Eigenschaften der Sedimente für chemische Substanzen ist - etwa im Bereich des Umweltschutzes - von großer Bedeutung. Um wirklichkeitsnahe Ergebnisse zu erreichen, ist es dabei von großer Bedeutung, eine Wasserströmung zu erzeugen, die definierte Bodenschubspannungen in der Sediment/Wasser-Grenzschicht verursacht.

Die Erzeugung einer relativ großflächigen Grenzschicht zwischen einem Substrat und einem Fluid mit definierter, insbesondere homogener Bodenschubspannungsverteilung kann weiter für industrielle Herstellungsverfahren, insbesondere mikrobiologische Herstellungsverfahren, Ausfällungsverfahren, Oberflächenvergütungsverfahren und dgl. von Bedeutung sein.

Der Erfindung liegt damit die Aufgabe zugrunde, ein Verfahren sowie eine Vorrichtung zu schaffen, die die Erzeugung definierter Bodenschubspannungen insbesondere in einer zu untersuchenden, in einen geschlossenen Raum eingebrachten Sediment/Wasser-Grenzschicht ermöglicht.

Erfindungsgemäß wird diese Aufgabe gelöst durch Erzeugen einer um die Achse des Raumes rotierenden Strömung des Fluids, bei dessen gleichzeitigem definierten Absaugen im Bereich der Rotationsachse der Strömung.

Die zur Lösung dieser Aufgabe vorgeschlagene Vorrichtung ist gekennzeichnet durch ein oben mit einem Deckel verschlossenes Rohr, ein unterhalb des Deckels koaxial zu dem Rohr angeordnetes Rührwerk, dessen Außendurchmesser kleiner ist als der Innendurchmesser des Rohres, einer den Deckel mittig durchdringenden, in diesem drehbar gelagerten, das Rührwerk antreibenden Hohlachse zum Abpumpen des Fluids aus dem von der Vorrichtung abgeschlossen Raum, wenigstens einer Rückführöffnung zum Ersetzen des durch die Hohlachse aus dem geschlossenen Raum abgepumpten Fluids und eine Pumpe zum Abpumpen des Fluids durch die Hohlachse.

Die Unteransprüche geben vorteilhafte Ausgestaltungen der erfindungsgemäß vorgeschlagenen Vorrichtung an.

Ausführungsbeispiele der Erfindung werden im folgenden anhand einer Zeichnung erläutert. Dabei zeigt:

Fig. 1 eine Querschnittsdarstellung durch ein erstes Ausführungsbeispiel, und

Fig. 2 eine Querschnittsdarstellung durch ein zweites Ausführungsbeispiel.

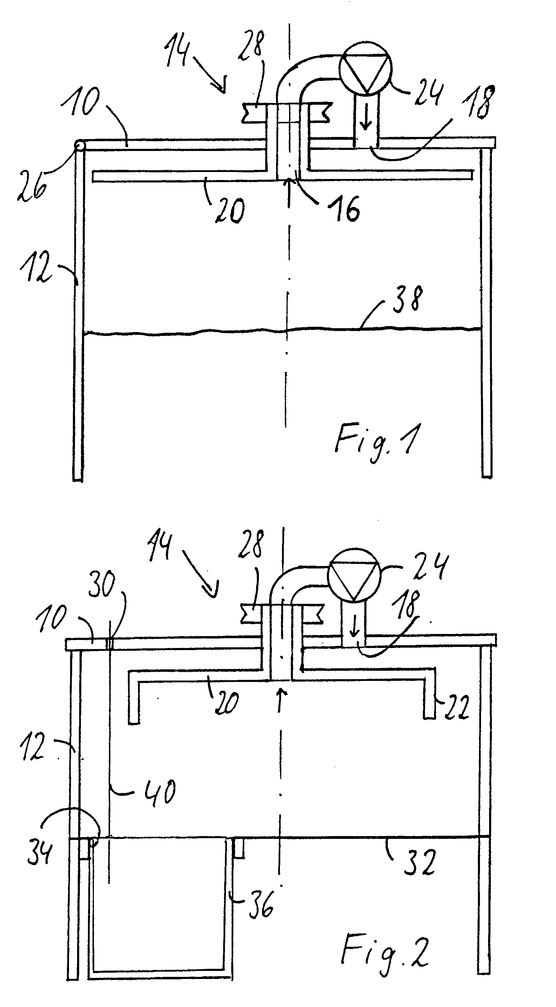

Die Vorrichtung nach Fig. 1 besteht aus einem unten offenen und oben von einem Deckel 10 verschlossenen Rohr 12, wobei der Deckel 10 mit dem Rohr 12 über ein Gelenk 26 verbunden ist und somit aufgeklappt werden kann.

Unterhalb des Deckels 10 ist ein Rührwerk 14 angeordnet, das aus einer kreisrunden Scheibe 20 besteht. Die Scheibe 20 wird von einer Hohlachse 16 getragen, die in dem Deckel 10 drehbar gelagert ist und über ein außerhalb des Deckels 10 angeordnetes Antriebsrad 28 angetrieben wird.

An die Hohlachse 16 ist ein Schlauch angekuppelt, der zu einer Pumpe 24 führt, von der wiederum ein weiterer Schlauch zu einer achsnah angeordneten Rückführöffnung 18 in dem Deckel 10 führt.

Bei Antrieb der Hohlachse 16 über das Antriebsrad 28 mittels eines - nicht gezeigten - Elektromotors wird das Rührwerk 14 in Drehung versetzt. Dies verursacht wiederum eine Strömung, die im wesentlichen um die Achse der Vorrichtung rotiert. Die Rotation des Rührwerks 14 verursacht eine im wesentlichen um die Achse der Vorrichtung rotierende Strömung des den Raum füllenden Wassers, wobei der Strömungsvektor von außen nach innen geringer wird. Das Rührwerk 14 verursacht so eine nicht homogene Schubspannung in der Wasser/Sediment-Grenzschicht, die entsprechend von außen nach innen abnimmt. Bei zusätzlichem Absaugen des Wassers durch die Hohlachse 16 wird eine weitere Strömungskomponente verursacht, die von außen nach innen zunimmt. Bei geeigneter Dimensionierung des Rohres 12 und des Rührwerks 14 sowie einer Anpassung der pro Zeiteinheit abgesaugten Wassermenge und der Umdrehungsgeschwindigkeit des Rührwerks 14 wird eine über im wesentlichen die ganze Querschnittsfläche der Vorrichtung dem Betrage nach (nicht also der Richtung nach!) homogene Strömung erreicht, die wiederum zu einem Feld homogener Geschwindigkeitsgradienten und damit zu einer homogenen Bodenschubspannung an der Sediment/Wasser-Grenzschicht der Vorrichtung führt.

Die bei dem in Fig. 2 gezeigten Ausführungsbeispiel dargestellte, parallel zu der Wandung des Rohres 12 aber mit einem deutlichen Abstand zu dieser angeordneten Schürze 22 (Fig. 2) macht es es möglich, am Rand der Scheibe Meßsonden durch Durchführungen 30 in dem Deckel 10 benachbart zu der Wandung des Rohres 12 in den von der Vorrichtung umschlossenen Raum einzuführen, um Messungen durchzuführen. Bei dieser Ausführungsform macht sich die Erfindung die Couette-Strömung zunutze, wodurch im wesentlichen derselbe Effekt erreicht wird, als wenn sich die Scheibe 20 bis an die Wandung des Rohres 12 erstreckt (Fig. 1).

Die Form der Scheibe ist den jeweiligen Gegebenheiten entsprechend zu wählen. So kann es erforderlich sein, daß die Scheibe sich nach außen hin verjüngend oder aber nach außen hin verdickend ausgebildet ist. Weiter kann das Rührwerk statt mit einer Scheibe mit sternförmig von der Hohlachse nach außen ragenden Sternen gebildet werden. Auch eine gelochte Scheibe oder aber eine mit einer Gitterstruktur versehene Scheibe ist möglich. Das Rührwerk kann auch glockenförmig ausgebildet sein.

Das Rohr 12 ist lediglich dann kreisrund auszubilden, wenn eine homogene Schubspannung in der Sediment/Wasser-Grenzschicht bewirkt werden soll. Wenn dagegen ein definiertes, aber nicht-homogenes Bodenschubspannungsfeld an der Sediment/Wasser-Grenzschicht aufgebaut werden soll, kommen auch andere Rohrquerschnitte, insbesondere elliptische Rohrquerschnitte, in Betracht.

Darüber hinaus können definierte nicht-homogene Bodenspannungsfelder auch durch entsprechende Entstellungen der pro Zeiteinheit abgepumpten Wasservoluminar oder der Umdrehungszahl des Rührwerks bewirkt werden.

Bei Verwendung des Gerätes als Laborsimulator ist er so auszubilden, daß er eine etwa 20 cm tiefe Sedimentfüllung mit überliegender Wassersäule aufnehmen kann. Weiter ist ein seitlich in das Rohr einschiebbarer Boden 32 vorgesehen, der entfernt werden kann, um Sedimentkerne auch direkt im Feld zu sammeln.

Bei Felduntersuchungen ist die Vorrichtung derart auszubilden, daß diese abgeworfen und auf den Meeresboden abgesenkt werden kann. In diesem Fall ist kein das Rohr 12 unten abschließender Boden 32 vorgesehen.

Die hier vorgeschlagene Vorrichtung kann sowohl für Untersuchungen der sich bei bestimmten, durch die Wahl der Rotationsgeschwindigkeit des Rührwerks 14 und der pro Zeiteinheit abgepumpten Wassermenge bewirkten Bodenschubspannungen an der Sediment/Wasser-Grenzschicht als auch an der sich dabei von dem Boden ablösenden Sediment-Agglomerationen dienen, indem in den Pumpkreislauf Mittel zum Auffangen dieser Sediment-Agglomerationen eingebracht werden. Derartige Mittel zum Auffangen von bei einer bestimmten Bodenschubspannung erodierten Sediment-Agglomerationen sind auch dann erforderlich, wenn es gilt, das verbleibende Sediment zu untersuchen.

In Fig. 2 ist ein Ausführungsbeispiel gezeigt, bei dem der Boden 32 des Gerätes mit einer Ausnehmung 34 versehen ist, die zur Aufnahme eines Sedimenteinsatzes 36 ausgebildet ist.

Der Durchmesser der Ausnehmung 34 ist damit kleiner als der Radius des Bodens 32. Bei einer derartigen Ausbildung wird es auf einfache Weise möglich, ein im Feld gewonnenes Sediment im Labor bzw. im Schiffslabor zu untersuchen.

Die vorgeschlagene Vorrichtung ist insbesondere für Untersuchungen an der Wasser/Sediment-Grenzschicht von Gewässern geeignet. Es kommen jedoch auch eine Vielzahl von weiteren Anwendungen in Betracht. Beispielsweise ist es bei mikrobiologischen Herstellungsverfahren erwünscht, daß Mikroorganismen, deren Stoffwechselprodukte gewonnen werden sollen, von einem Fluid mit einer steuerbaren Strömungsgeschwindigkeit überströmt werden. In Betracht kommt weiter eine Anwendung in der industriellen Verfahrenstechnik insbesondere dort, wo ein definierter vertikaler Austausch zwischen einem Fluid und einem Substrat erreicht werden soll. Dies ist beispielsweise für Oberflächenvergütungen von Bedeutung.

Bei dem Fluid braucht es sich nicht um Wasser zu handeln, vielmehr kann es sich auch um Luft oder um andere, beispielsweise atomisierte Metalle tragende Gase handeln.

**Patentansprüche**

1. Verfahren zum Erzeugen von definierten Bodenschubspannungen an einer in einen geschlossenen Raum eingebrachten Substrat/Fluid-Grenzschicht, gekennzeichnet durch Erzeugen einer um die Achse des Raumes rotierenden Fluidströmung bei gleichzeitigem definierten Absaugen des Fluids im Bereich der Rotationsachse der Strömung.

2. Verfahren nach Anspruch 1, dadurch gekennzeichnet, daß es sich bei dem Substrat um ein Sediment und bei dem Fluid um Wasser handelt, wobei die Sediment/WasserGrenzschicht auf physikalische, chemische und/oder biologische Zustände und/oder Veränderungen hin untersucht werden soll.

3. Vorrichtung zum Erzeugen von definierten Bodenschubspannungen an einer in einen geschlossenen Raum eingebrachten Substrat/Fluid-Grenzschicht, insbesondere an einer zu untersuchenden Sediment/Wasser-Grenzschicht, gekennzeichnet durch

   - ein oben mit einem Deckel (10) verschlossenes Rohr (12),
   - ein unterhalb des Deckels (10) koaxial zu dem Rohr (12) angeordnetes Rührwerk (14), dessen Außendurchmesser kleiner ist als der Innendurchmesser des Rohres (12),

- einer den Deckel (10) mittig durchdringenden, in diesem drehbar gelagerten, das Rührwerk (14) antreibenden Hohlachse (16) zum Abpumpen von Wasser aus dem von der Vorrichtung umschlossenen Raum,
- wenigstens einer Rückführöffnung (18) zum Ersetzen des durch die Hohlachse (16) aus dem umschlossenen Raum abgepumpten Wassers, und
- eine Pumpe (24) zum Abpumpen des Wassers durch die Hohlachse (16).

4. Vorrichtung nach Anspruch 3, dadurch gekennzeichnet, daß das Rührwerk (14) als eine Scheibe (20) ausgebildet ist.

5. Vorrichtung nach Anspruch 4, dadurch gekennzeichnet, daß die Scheibe profiliert ausgebildet ist.

6. Vorrichtung nach Anspruch 4, dadurch gekennzeichnet, daß die Scheibe gelocht ausgebildet ist.

7. Vorrichtung nach Anspruch 4, dadurch gekennzeichnet, daß die Scheibe durch eine Gitterstruktur gebildet wird.

8. Vorrichtung nach Anspruch 3, dadurch gekennzeichnet, daß das Rührwerk (14) eine Vielzahl von sternförmig um die Hohlachse (16) angeordnete Flügel aufweist.

9. Vorrichtung nach Anspruch 3, dadurch gekennzeichnet, daß das Rührwerk (14) glockenförmig ausgebildet ist.

10. Vorrichtung nach einem der Ansprüche 3 bis 9, dadurch gekennzeichnet, daß das Rührwerk (14) topfförmig mit einer parallel und mit Abstand zur Wandung des Rohres (12) umlaufenden Schürze (22) ausgebildet ist.

11. Vorrichtung nach einem der Ansprüche 3 bis 10, dadurch gekennzeichnet, daß die Hohlachse (16) und die Rückführöffnung(en) (18) über die Pumpe (24) miteinander verbunden sind.

12. Vorrichtung nach einem der Ansprüche 3 bis 11, dadurch gekennzeichnet, daß der Deckel (10) mit dem Rohr (12) über ein Gelenk (26) oder dgl. verbunden ist.

13. Vorrichtung nach einem der Ansprüche 3 bis 12, dadurch gekennzeichnet, daß das Rohr (12) im Querschnitt kreisrund ist.

14. Vorrichtung nach einem der Ansprüche 3 bis 12, dadurch gekennzeichnet, daß das Rohr (12) im Querschnitt elliptisch ist.

15. Vorrichtung nach einem der Ansprüche 3 bis 14, dadurch gekennzeichnet, daß das Rohr (12) für Felduntersuchungen unten offen ausgebildet ist.

16. Vorrichtung nach einem der Ansprüche 3 bis 15, dadurch gekennzeichnet, daß die Vorrichtung für Laboruntersuchungen mit einem das Rohr (12) unten abschließenden Boden (32) versehen ist.

17. Vorrichtung nach Anspruch 16, dadurch gekennzeichnet, daß der Boden (32) seitlich in das Rohr (12) einschiebbar ausgebildet ist.

18. Vorrichtung nach Anspruch 16, dadurch gekennzeichnet, daß der Boden (32) mit einer exzentrisch angeordneten Ausnehmung (34) zur Aufnahme eines von unten an den Boden (32) anzusetzenden, mit dieser bündig abschließenden Sedimenteinsatzes (36) versehen ist.

19. Vorrichtung nach Anspruch 18, dadurch gekennzeichnet, daß die Ausnehmung (34) mit einem Bajonettverschluß versehen ist.

20. Vorrichtung nach einem der Ansprüche 3 bis 19, dadurch gekennzeichnet, daß Mittel zum Auffangen von abgesaugten Feststoffen, insbesondere Sediment-Agglomerationen, vorgesehen sind.

**Claims**

1. Method for the generation of defined wall shearing stresses at a substrate/fluid interface brought into a confined space, characterized by generating a fluid flow moving rotationally around the axis of the space under simultaneous metered sucking off of the fluid in the area of the rotational-flow axis.

2. Method according to claim 1, characterized in that the substrate is a sediment and the fluid is water, whereby the boundary layer is to be investigated in respect to physical, chemical, and/or biological conditions and/or changes.

3. Device for the generation of defined wall shearing stresses at a substrate/fluid boundary layer in a confined space, especially over a sediment/water boundary layer to be investigated, characterized by

- a tube (12) sealed on top by a lid (10),
- a stirrer (14) positioned below the lid (10) coaxially to the tube (12), the outer diameter of which is smaller than the inner diameter of the tube (12),
- an axle (16) centrally penetrating and freely rotating in the lid (10), which drives the stirrer (14) and is hollow to pump the water out of the confined space,
- at least one return opening (18) to replace the water pumped out of the confined space through the hollow axle (16), and
- a pump (24) to pump out the water through the hollow axle (16).

4. Device according to claim 3, characterized in that the stirrer (14) is shaped as a flat disk (20).

5. Device according to claim 4, characterized in that the disk is profiled.

6. Device according to claim 4, characterized in that the disk is perforated.

7. Device according to claim 4, characterized in that the disk is formed by a grid structure.

8. Device according to claim 3, characterized in that the stirrer (14) is comprised of a multitude of wings attached radially to the hollow axle (16).

9. Device according to claim 3, characterized in that the stirrer (14) is shaped bell-like.

10. Device according to one of the claims 3 to 9, characterized in that the stirrer (14) is shaped pot-like with an attached skirt (22) aligned parallel with and spaced from the wall of the tube (12).

11. Device according to one of the claims 3 to 10, characterized in that the the hollow axle (16) and the return opening(s) (18) are connected by said pump (24).

12. Device according to one of the claims 3 to 11, characterized in that said lid (10) is connected to the tube (12) via a hinge (26) or similar joint.

13. Device according to one of the claims 3 to 12, characterized in that the tube (12) has a circular cross section.

14. Device according to one of the claims 3 to 12, characterized in that the tube (12) has an elliptic cross section.

15. Device according to one of the claims 3 to 14, characterized in that the tube (12) is open at the bottom for field investigations.

16. Device according to one of the claims 3 to 15, characterized in that the tube (12) is equipped with a sealing bottom (32) for laboratory investigations.

17. Device according to claim 16, characterized in that the bottom (32) can be pushed sidewise into the tube (12).

18. Device according to claim 16, characterized in that the bottom (32) is equipped with an eccentrically located pocket (34) to receive a sediment insert (36) to be attached from beneath and flush with the bottom (32).

19. Device according to claim 18, characterized in that the pocket (34) is equipped with a fast-lock connector.

20. Device according to one of the claims 3 to 19, characterized in that means are provided for collecting sucked off particles, especially sediment aggregates.

## Revendications

1. Procédé de génération d'efforts définis de cisaillement du sol à la couche limite substrat/fluide amenée dans un espace fermé, caractérisé par la génération d'un courant fluide tournant autour de l'axe de l'espace et l'aspiration simultanée et définie du fluide dans la région de l'axe de rotation.

2. Procédé suivant la revendication 1, caractérisé par le fait que le substrat est un sédiment et que le fluide est de l'eau et que la couche limite sédiment/eau doit être analysée pour des états et/ou des changements physiques, chimiques et/ou biologiques.

3. Dispositif de génération d'efforts définis de cisaillement du sol à la couche limite substrat/fluide amenée dans un espace fermé, spécialement à une couche limite sédiment/eau à examiner, caractérisé par

- un tube (12) fermé en haut par un couvercle (10) ;
- un dispositif agitateur (14) arrangé en dessous du couvercle (10) et coaxialement au tube (12) et dont le diamètre extérieur est plus petit que le diamètre intérieur du tube (12) ;

- un arbre creux (16) pénétrant centriquement le couvercle dans lequel il est logé de façon rotative et commandant le dispositif agitateur (14), et qui servira à pomper de l'eau de l'espace entouré par le dispositif ;

- au moins une ouverture de recyclage (18) pour remplacer l'eau pompée de l'espace entouré à travers l'arbre creux (16) ;

- une pompe (24) pour pomper de l'eau à travers l'arbre creux (16).

4. Dispositif suivant la revendication 3, caractérisé par le fait que le dispositif agitateur (14) est construit en forme de disque.

5. Dispositif suivant la revendication 4, caractérisé par le fait que le disque est profilé.

6. Dispositif suivant la revendication 4, caractérisé par le fait que le disque est perforé.

7. Dispositif suivant la revendication 4, caractérisé par le fait que le disque est formé par une structure de grille.

8. Dispositif suivant la revendication 3, caractérisé par le fait que le dispositif agitateur (14) présente une multiplicité d'ailes qui sont arrangées en étoile autour de l'arbre creux.

9. Dispositif suivant la revendication 3, caractérisé par le fait que le dispositif agitateur (14) est construit en forme de cloche.

10. Dispositif suivant l'une des revendications 3 à 9, caractérisé par le fait que le dispositif agitateur (14) est fait en forme de pot et muni d'un tablier (22) tournant parallèlement et avec écart autour de la paroi du tube (12).

11. Dispositif suivant l'une des revendications 3 à 10, caractérisé par le fait que l'arbre creux (16) et la/les ouverture(s) de recyclage (18) sont liés par la pompe (24).

12. Dispositif suivant l'une des revendications 3 à 11, caractérisé par le fait que le couvercle est lié au tube (12) par une articulation (26) ou analogue.

13. Dispositif suivant l'une des revendications 3 à 12, cacactérisé par le fait que le tube (12) est rond vu en coupe transversale.

14. Dispositif suivant l'une des revendications 3 à 12, caractérisé par le fait que le tube (12) est elliptique vu en coupe transversale.

15. Dispositif suivant l'une des revendications 3 à 14, caractérisé par le fait que le tube est ouvert en bas pour des analyses de champ.

16. Dispositif suivant l'une des revendications 3 à 15, caractérisé par le fait que le dispositif pour des analyses de laboratoire est muni d'un fond (32) fermant le tube (12) en bas.

17. Dispositif suivant la revendication 16, caractérisé par le fait que le fond (32) est latéralement embrayable.

18. Dispositif suivant la revendication 16, caractérisé par le fait que le fond (32) présente un trou (34) arrangé excentriquement pour recevoir une cage à sédiments (36) appliquée par en bas au fond et formant une surface plane avec le trou.

19. Dispositif suivant la revendication 18, caractérisé par le fait que le trou (34) est muni d'une fermeture à baïonnette.

20. Dispositif suivant l'une des revendications 3 à 19, caractérisé par le fait que des moyens pour recueillir des solides aspirés, spécialement des agglomérations du sédiment, sont prévus.

EP 0 292 828 B1

Fig.1

Fig.2

7